(19) Europäisches Patentamt / European Patent Office / Office européen des brevets

(11) **EP 1 801 101 B1**

(12) **EUROPEAN PATENT SPECIFICATION**

(45) Date of publication and mention
of the grant of the patent:
**14.01.2009 Bulletin 2009/03**

(51) Int Cl.:
***C07D 201/12*** *(2006.01)* ***C08J 11/24*** *(2006.01)*

(21) Application number: **06024522.2**

(22) Date of filing: **27.11.2006**

(54) **Method of depolymerizing polyamides and method of manufacturing polyamide monomers**

Verfahren zur Depolymerisation von Polyamiden und Verfahren zur Herstellung von
Polyamidmonomeren

Procédé de dépolymérisation de polyamides et procédé de préparation des monomères de polyamide

(84) Designated Contracting States:
**ES**

(30) Priority: **25.11.2005 JP 2005339619
28.02.2006 JP 2006053153**

(43) Date of publication of application:
**27.06.2007 Bulletin 2007/26**

(73) Proprietors:
• **Yamaguchi University
Yamaguchi 753-8511 (JP)**
• **Ube Industries, Ltd.
Ube-shi
Yamaguchi 755-8633 (JP)**

(72) Inventors:
• **Kamimura, Akio
2-chome
Ube-shi
Yamaguchi 755-8611 (JP)**
• **Sugimoto, Tsunemi
Ube-shi,
Yamaguchi 755-8633 (JP)**
• **Kaiso, Kouji
Ube-shi,
Yamaguchi 755-8633 (JP)**

(74) Representative: **Bublak, Wolfgang
European Patent Attorney
Bardehle, Pagenberg, Dost, Altenburg, Geissler
Postfach 86 06 20
81633 München (DE)**

(56) References cited:
EP-A1- 0 891 969          WO-A-97/49652
DE-B- 1 235 924          US-A- 2 840 606
US-A1- 2002 037 939          US-A1- 2002 169 223

• DATABASE WPI Week 200258 Derwent
Publications Ltd., London, GB; AN 2002-542276
XP002431353 & JP 2002 148253 A (SUMIKA
BUNSEKI CENTER KK) 22 May 2002 (2002-05-22)
• DATABASE WPI Week 200056 Derwent
Publications Ltd., London, GB; AN 2000-589747
XP002431333 & JP 2000 191638 A (KOBE STEEL
LTD) 11 July 2000 (2000-07-11)

**Description**

The present invention relates to a method of depolymerizing polyamides such as nylon 6 and a method of manufacturing polyamide monomers.

Polyamides, typically nylon fibers, have been employed in various uses such as films and engineering plastics and, in recent years, reductions in wastes, effective use of resources and environmental preservations have been taken seriously. Thus, polyamide products are recycled.

As a method of depolymerizing polyamides or a method of manufacturing polyamide monomers, for example. Patent Document 1 (JP 2000-191638 A) describes a method of depolymerizing an ε-caprolactam oligomer into an ε-caprolactam, which method comprises bringing a high-temperature, high-pressure water into contact with the oligomer at 280-450 °C under 100-500 kg/cm$^2$.

A depolymerization reaction of the polyamide with the high-temperature, high-pressure water carried out as described in Patent Document 1, however, may corrode the interior of a metal device in a relatively short period of time.

US-A-2 840 606 describes an alkaline hydrolysis of a polyamide characterized by separation into diacid and diamine components upon alkaline hydrolysis. EP-A-0 891 969 relates to a process for depolymerising one or more polyamides in the presence of at least an aliphatic alcohol, the depolymerisation taking place with at most 40 mol.% catalyst. US -A-2002/037939 discloses a process for recovering polyamide material from post-industrial and post-consumer products containing the polyamide material and insoluble materials. JP-A-2002-148253 discloses a method for the analysis of polyamides using a lower C1-3 alcohol such as ethanol in supercritical state.

It is the object of the present invention to provide a method of depolymerizing polyamides and a method of manufacturing polyamide monomers capable of carrying out a depolymerization reaction of a polyamide without causing any corrosion inside a metal device.

This object has been achieved by the surprising finding that a reaction of an alcohol having two or more carbon atoms with a polyamide makes it possible to carry out a depolymerization reaction of the polyamide at a high monomer recovery rate without causing any corrosion inside a metal device. Thus, the present invention provides a method of depolymerizing polyamides, comprising reacting an alcohol having two or more carbon atoms with a polyamide to carry out a depolymerization reaction of the polyamide. It also provides a method of manufacturing po lyamide monomers, comprising reacting an alcohol having two or more carbon atoms with a polyamide to yield a monomer of the polyamide. As described above, the present invention makes it possible to provide a method of depolymerizing polyamides and a method of manufacturing polyamide monomers capable of carrying out a depolymerization reaction of a polyamide through a reaction of an alcohol having two or more carbon atoms with the polyamide without causing any corrosion inside a metal device.

The most important characteristic of the present invention lies in the use of an alcohol having two or more carbon atoms. Examples of the alcohol for use in the method of depolymerizing polyamides and the method of manufacturing polyamide monomers according to the present invention may include ethanol, 1-propanol (n-propanol), 2-propanol, (isopropanol), aryl alcohol, 1-butanol (n-butanol), 2-butanol (sec-butanol), 2-methyl-1-propanol (isobutanol), 2-methyl-2-propanol (t-butanol). 3-butene-2-ol, crotyl alcohol, cyclopropane methanol, 3-butene-1-ol, 2-methyl-2-propene-1-ol, 3-butyne-1-ol, 2-butyne-1-ol, 3-butyne-2-ol, 1-pentanol (n-pentanol), 2-pentanol (sec-amyl alcohol), 3-pentanol, 2-methyl-1-butanol, 2-methyl-2-butanol, 3-methyl-2-butanol, 3-methyl-1-butanol, 2,2-dimethyl-1-propanol (t-amylalcohol), 1-cyclopropyl ethanol, 1-pentene-3-ol, 4-pentene-2-ol. 4-pentene-1-ol, 3-pentene-2-ol, 3-methyl-3-butene-1-ol, 2-methyl-3-butene-2-ol, 3-methyl-2-butene-1-ol, cyclobutane methanol, 2-methyl cyclopropane methanol, 2-methyl-3-butene-1-ol, 2-methyl-3-butyne-2-ol, 2-pentyne-1-ol, 4-pentyne-2-ol, 4-pentyne-1-ol, 1,4-pentadiene-3-ol, 2-pentyne-1-ol, 1-hexanol (n-hexanol), 2-hexanol, 3-hexanol, 3-methyl-3-pentanol, 4-methyl-1-pentanol, 4-methyl-2-pentanol, 3-methyl-1-pentanol, 2-methyl-2-pentanol, 3-methyl-2-pentanol, 2-methyl-3-pentanol, 2-methyl-1-pentanol, 2-ethyl-1-butanol, 2,3-dimethyl-2-butanol, 3.3-dimethyl-2-butanol, 3,3-dimethyl-1-butanol, cyclohexanol, 1-heptanol (n-heptanol), 2-heptanol, 3-heptanol, 2-methyl-3-hexanol, 2-methyl-2-hexanol, 5-methyl-1-hexanol, 5-methyl-1-hexanol, 2,2-dimethyl-3-pentanol, 3-ethyl-3-pentanol, 2,3-dimethyl-3-pentanol, 2,4-dimethyl-3-pentanol, 4,4-dimethyl-2-pentanol, 1-octanol (n-octanol), 2-octanol, 3-octanol, 6-methyl-2-heptanol, 4-methyl-3-heptanol, 2-ethyl-1-hexanol. 2,4,4-trimethyl-1-pentanol, 2-propyl-1-pentanol, 1-nonanol, 2-nonanol, 3-methyl-3-octanol, 2,6-dimethyl-4-heptanol, 3,5,5-trimethyl-1-hexanol, 3-ethyl-2,2-dimethyl-3-pentanol, 1-decanol (n-decanol), 2-decanol, 3,7-dimethyl-1-octanol, 3,7-dimethyl-3-octanol, 1-undecanol, 2-undecanol, 1-dodecanol (n-dodecanol), 2-dodecanol, 2-butyl-1-octanol, cyclododecanol, 1-tridecanol, 1-tetradecanol, 2-tetradecanol,1-pentadecanol,1-hexadecanol, 2-hexadecanol, 2-hexyl-1-decanol, 1-heptadecanol, and 1-octadecanol. Among those, in the case of a secondary or tertiary alcohol, the alcohol may have any carbon atoms not limited particularly, with a very excellent monomer recovery and a less impurity production. In the case of a primary alcohol, the alcohol may have any carbon atoms not limited particularly though four or more carbon atoms are particularly preferable. In this case, it is not comparable with the secondary or tertiary alcohol but results in a very excellent monomer recovery and a less impurity production in comparison with the use of methanol. Preferably, the alcohol is a cyclic alcohol. The cyclic alcohol as recited in the present invention means an alcohol having a cyclic carbon chain. For example, it may include cyclohexanol and cyclododecanol. Further, an alcohol having 3-6 carbon atoms may be used preferably because it has a large reaction speed of depolymerization.

These alcohols are subjected to reactions preferably in a liquid phase, a subcritical state or a supercritical state, more particularly in a supercritical state. The subcritical state as recited in the present invention means a state having a temperature or pressure kept at near a critical point, slightly lower than the critical point. The reaction pressure is around 8-40 MPaG (G denotes gauge pressure). For example, the supercritical state can be achieved through heating and pressurizing, or heating in a tightly closed state. The depolymerization of a polyamide according to the present invention may be achieved through mixing the polyamide with an alcohol and then heating the alcohol into the supercritical state, or adding an alcohol in the supercritical state to the polyamide. As shown in Table 1, major alcohols have respective critical temperatures and critical pressures. The reaction at near the supercritical state may preferably include a reaction at least at a high temperature, for example, a reaction with an alcohol at a reaction temperature of 250-450 °C, preferably 300 °C or higher, more preferably 350 °C or higher. Also in this case, a high-temperature alcohol may be reacted with or a reacted alcohol may be heated up to a high-temperature state.

[Table 1]

|  | Critical Temperature (°C) | Critical Pressure (MPaG) |
|---|---|---|
| Ethanol | 243 | 6.4 |
| 1-Propanol | 264 | 5.2 |
| 2-Propanol | 253 | 4.8 |
| 1-Butanol | 290 | 4.4 |
| 2-Methyl-1-propanol | 275 | 4.3 |
| 2-Butanol | 263 | 4.2 |
| 2-Methyl-2-propanol | 233 | 4.0 |
| 1-Pentanol | 313 | 3.8 |
| 2.2-Dimethyl-1-propanol | 276 | 4.0 |
| 1-Hexanol | 337 | 3.5 |
| Cyclohexanol | 352 | 3.8 |
| 1-Heptanol | 360 | 3.0 |
| 1-Octanol | 385 | 2.7 |
| 1-Decanol | 414 | 2.4 |
| 1-Dodecanol | 406 | 1.9 |

The method of depolymerizing polyamides according to the present invention is aimed at a polyamide, which is a polymer having two or more amide (-C(O)NH-) bonds, More specifically, it may be a polymer including a diamine and a dicarboxylic acid chained through an amide bond. Alternatively, it may be a chain polymer resulted from a ring-opening polymerization of a monomer such as a caprolactam, which is shaped as cyclized through dehydration condensation of an amino group and a carboxyl group in one molecule. The degree of polymerization is not limited particularly, and an oligomer, which is a lower polymer, may be available. The polyamide may include one type or two or more types in mixture. Examples include nylon 6, nylon 66, nylon 11 and nylon 12. Among those, nylon 6 is particularly preferable. More specifically, it may include a waste of nylon 6 fiber carpets and an out-of-tolerance product containing an oligomer produced when a caprolactam is polymerized to manufacture nylon 6. As for the size of the polyamide, a pellet-shaped one having the longest portion of 10 mm or below is preferably used. A smaller diameter polyamide, for example, a powdered one can serve depolymerization as it is. When the polyamide has a shape of mass or elongated line, it is preferable to previously pulverize it using a pulverizer before depolymerization.

A reaction time in the method of depolymerizing polyamides according to the present invention is preferably 5 minutes to 4 hours, more preferably 0.5 hour to 4 hours. A ratio of a polyamide subjected to depolymerization to a total weight of the polyamide and an alcohol reacted therewith is preferably above zero but not larger than 50 % by weight, more preferably 5-15 % by weight, and most preferably 7-13 % by weight.

The method of depolymerizing polyamides according to the present invention is carried out without the use of a catalyst. Therefore, not only the reaction simply proceeds but also the method is excellent in cost because there is no need for a special device to use a catalyst.

The method or depolymerizing polyamides according to the present invention is considered as carried out through an intramolcular exchange reaction in which an amino group in a polyamide nucleophilically attacks an adjacent carbonyl carbon such that monomers dissociate sequentially. A reaction device for use in depolymerization of a polyamide may comprise an agitator-equipped cylindrical cell or a reaction tube. A reaction type may be either a continuous system or a batch system. A polyamide and an alcohol may be supplied separately into the reaction device though a previously prepared mixture may be supplied into the reaction device.

The monomer depolymerized through the method of depolymerizing polyamides according to the present invention may be purified either through dealcohol and separation steps or through only a dealcohol step. The products after the dealcohol step are separated into the monomer and heavier fractions and collected at a separation and collection step.

The dealcohol step is carried out in a single distillation device; a flash separation device composed of a flash drum and so forth; a (reduced-pressure) distillation device such as a distillation tower; an adsorption device such as an adsorption tower; or a drying device and the like. The separation and collection step is carried out in a single distillation device; a (reduced-pressure) distillation device such as a distillation tower; a thin film evaporation device; or devices for degassing, extraction, centrifugal separation, centrifugal settling, liquid cyclone, stationary separation, filtration, compression, and division. These dealcohol and separation steps may be carried out in a depressurized or pressurized state. The collected monomer is purified as it is or through a reduced-pressure distillation or recrystallization process or a sublimation means and then used for producing another polyamide.

The vaporized alcohol separated with the use of the flash separation device or the like in the dealcohol step is then condensed at a heat exchanger. The condensed alcohol is recycled at a pump for another depolymerization reaction to reduce the usage of the alcohol. When the vaporized alcohol is condensed at the heat exchanger, the alcohol or the polyamide-alcohol mixture fed to the depolymerization reaction may be used as a coolant in the heat exchanger. This enables the alcohol or the polyamide-alcohol mixture fed to the depolymerization reaction to be heated previously to reduce the thermal energy cost greatly.

A pump or the like may be located to re turn heavier fractions from the separation and collection step to a residue supply line. The heavier fractions include an oligomer and so forth and accordingly they can improve the monomer recovery when supplied to the depolymerization reaction again.

EXAMPLES

Examples of the method of depolymerizing polyamides and method of manufacturing polyamide monomers according to the present invention will now be described below. First, as Examples 1-16, into a 10 mL volume (7.53 mm inner diameter and 225 mm long) reaction tube of SUS 316 (stainless steel), 0.3 g of nylon 6 (Nylon chip 10223 from Ube Industries, Ltd. (2.4 mm diameter and 2.7 mm long chip)), and 4 g of an alcohol shown in Table 2 were fed. The air in a dead space inside the reaction tube was replaced with argon and then sealed. The sealed reaction tube was located stationary for 1.5 hours in an electric furnace heated up to 370 °C. Thereafter, the reaction tube was extracted from the electric furnace and immersed into water to cool it down to the atmospheric temperature. The reaction pressure was measured using a diaphragm pressure gauge connected to one end of the reaction tube via a metallic joint. When the alcohol is ethanol, 2-propanol, and 2-methyl-2-propanol, the reaction pressure was 26.7, 22.1, and 19.6 MPaG, respectively. The reaction pressure was also measured on other alcohols through the same method as described above. The reaction temperature was measured with a thermometer inserted through the other end of the reaction tube provided with the diaphragm pressure gauge. The small inner volume of the reaction tube consequently makes no difference between the temperature in the heated electric furnace and the temperature inside the reaction tube. The amount of water contained in the alcohol to be used was measured with a micro moisture meter (AQ7 from Hiranuma Sangyo Co., Ltd.). When the alcohol is solid at the atmospheric temperature, the solid is directly thrown into the meter to measure the amount of water. The results are shown in Table 2.

[Table 2]

|  | Alcohol | Water (ppm) | Recovery of ε-Caprolactam (%) |
|---|---|---|---|
| Example 1 | Ethanol | 545 | 36.18 |
| Example 2 | 1-Propanol | 270 | 35.3 |
| Example 3 | 2-Propanol | 225 | 90.03 |
| Example 4 | 1-Butanol | 250 | 47.05 |
| Example 5 | 2-Methyl-1-prop anol | 176 | 44.26 |

(continued)

| | Alcohol | Water (ppm) | Recovery of ε-Caprolactam (%) |
|---|---|---|---|
| Example 6 | 2-Butanol | 93 | 96.1 |
| Example 7 | 2-Methyl-2-prop anol | 147 | 94.86 |
| Example 8 | 1-Pentanol | 340 | 41.61 |
| Example 9 | 2,2-Dimethyl-1-propanol | 2200 | 97.24 |
| Example 10 | 1-Hexanol | 414 | 45.6 |
| Example 11 | Cyclohexanol | 504 | 95.4 |
| Example 12 | 1-Heptanol | 280 | 54.39 |
| Example 13 | 1-Octanol | 70 | 54.79 |
| Example 14 | 1-Decanol | 1800 | 55.31 |
| Example 15 | 1-Dodecanol | 214 | 61.36 |
| Example 16 | Cyclododecanol | 237 | 83.4 |
| Comparative Example | Methanol | 1600 | 13.9 |

As a comparative example, depolymerization of nylon 6 was executed like Example 1 except for a reaction of methanol.

The reacted mixtures depolymerized in Examples 1-16 and Comparative example were each extracted from the reaction tube for quantification of ε-caprolactam. The quantification of ε-caprolactam was carried out using a gas chromatograph (GC-14B from Shimadzu Corporation). The recovery of ε-caprolactam was calculated based on the following expression 1. The colors of the reacted mixtures were also observed. The results are shown in Table 2.

[Expression 1]

Recovery of ε-Caprolactam (%) =

[(ε-Caprolactam in Reacted Mixture (Weight %)) × (Total Mass

of Nylon 6 and Solvent and Catalyst before Reaction (g))] / (Mass

of Nylon 6 before Reaction (g)) × 100

In this case, Mass of Catalyst is set at zero (g) when no catalyst is used.

As shown in Table 2, nylon 6 can be depolymerized through a reaction with an alcohol having two or more carbon atoms. When the reaction tube was torn after the reaction to visually observe the inner wall of the reaction tube, it was confirmed that the luster of stainless steel could be retained in any one of Examples 1-16. It was found that Examples 1-16 have higher recoveries of ε-caprolactam than that of the methanol-reacted comparative example.

Except that the alcohol is unchanged from 2-propanol (with a water concentration of 225 ppm) and the reaction temperature and time were varied, the same method as in Examples 1-16 was employed to carry out depolymerization of nylon 6. The recoveries of ε-caprolactam were obtained in accordance with the above expression 1. The results are shown in Table 3. According to Table 3, the recoveries of ε-caprolactam at reaction temperatures of 350 °C and 370°C are greatly improved over those at 300 °C and 330 °C. This made it clear that the ε-caprolactam can be recovered with a particularly high yield at the reaction temperature of 350 °C or higher.

[Table 3]

| | Reaction Temperature (°C) | Reaction Time (Hours) | Recovery of ε-caprolactam (%) |
|---|---|---|---|
| Example 17 | 300 | 3 | 33.5 |
| Example 18 | 300 | 4 | 27.0 |
| Example 19 | 300 | 5 | 25.8 |

(continued)

| | Reaction Temperature (°C) | Reaction Time (Hours) | Recovery of $\varepsilon$-caprolactam (%) |
|---|---|---|---|
| Example 20 | 300 | 6 | 33.0 |
| Example 21 | 330 | 2 | 52.4 |
| Example 22 | 330 | 3 | 62.0 |
| Example 23 | 330 | 4 | 63.1 |
| Example 24 | 330 | 5 | 62.9 |
| Example 25 | 330 | 6 | 64.1 |
| Example 26 | 350 | 1 | 73.0 |
| Example 27 | 350 | 2 | 89.0 |
| Example 28 | 350 | 3 | 88.8 |
| Example 3 | 370 | 1.5 | 90.03 |
| Example 29 | 370 | 3 | 91.5 |

**Claims**

1. A method of manufacturing polyamide monomers, comprising reacting an alcohol having two or more carbon atoms with a polyamide to yield a monomer of the polyamide, wherein the reaction is carried out without catalyst, and wherein the reaction of the alcohol is carried out at 350°C or higher.

2. The method of manufacturing polyamide monomers according to claim 1, wherein the alcohol is a secondary or tertiary alcohol.

3. The method of manufacturing polyamide monomers according to claim 1 or 2, wherein the alcohol is an alcohol having four or more carbon atoms.

4. The method of manufacturing polyamide monomers according to any one of claims 1-3, wherein the alcohol is a cyclic alcohol.

5. The method of manufacturing polyamide monomers according to any one of claims 1-4, wherein the alcohol is reacted in a supercritical state.

**Patentansprüche**

1. Verfahren zur Herstellung von Polyamidmonomeren, umfassend das Umsetzen eines Alkohols mit zwei oder mehr Kohlenstoffatomen mit einem Polyamid unter Erhalt eines Monomers des Polyamids, wobei die Umsetzung ohne Katalysator durchgeführt wird, und wobei die Umsetzung des Alkohols bei 350°C oder höher durchgeführt wird.

2. Verfahren zur Herstellung von Polyamidmonomeren nach Anspruch 1, wobei es sich bei dem Alkohol um einen sekundären oder tertiären Alkohol handelt.

3. Verfahren zur Herstellung von Polyamidmonomeren nach Anspruch 1 oder 2, wobei es sich bei dem Alkohol um einen Alkohol mit vier oder mehr Kohlenstoffatomen handelt.

4. Verfahren zur Herstellung von Polyamidmonomeren nach einem der Ansprüche 1-3, wobei es sich bei dem Alkohol um einen cyclischen Alkohol handelt.

5. Verfahren zur Herstellung von Polyamidmonomeren nach einem der Ansprüche 1-4, wobei der Alkohol in einem superkritischen Zustand umgesetzt wird.

**Revendications**

1. Un procédé de fabrication de monomères de polyamide, comprenant la mise en réaction d'un alcool comportant deux atomes de carbone ou plus avec un polyamide pour donner un monomère du polyamide, dans lequel la réaction est opérée sans catalyseur, et dans lequel la réaction de l'alcool est opérée à 350°C ou plus.

2. Le procédé de fabrication de monomères de polyamide selon la revendication 1, dans lequel l'alcool est un alcool secondaire ou tertiaire.

3. Le procédé de fabrication de monomères de polyamide selon la revendication 1 ou 2, dans lequel l'alcool est un alcool ayant quatre atomes de carbone ou plus.

4. Le procédé de fabrication de monomères de polyamide selon l'une des revendications 1 à 3, dans lequel l'alcool est un alcool cyclique.

5. Le procédé de fabrication de monomères de polyamide selon l'une des revendications 1 à 4, dans lequel l'alcool est mis en réaction dans un état supercritique.

**EP 1 801 101 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000191638 A
- US 2840606 A
- EP 0891969 A
- US 2002037939 A
- JP 2002148253 A